# EUROPEAN PATENT APPLICATION

(11) **EP 4 535 122 A1**
(43) Date of publication of application: **09.04.2025**
(21) Application number: 23839787.1
(22) Date of filing: 22.05.2023
(51) Int. Cl.: G06F 1/16, H05K 1/14, H05K 9/00, A61B 5/05, H05K 3/12

(54) **WEARABLE DEVICE COMPRISING PRINTED CIRCUIT BOARD INCLUDING CONDUCTIVE PATTERN, AND ELECTRONIC DEVICE**

(30) Priority: 11.07.2022 KR 20220085340; 09.08.2022 KR 20220099485
(71) Applicant: Samsung Electronics Co., Ltd., Suwon-si, Gyeonggi-do 16677 (KR)
(72) Inventor: SHIN, Yongjoo, Suwon-si Gyeonggi-do 16677 (KR); KIM, Seho, Suwon-si Gyeonggi-do 16677 (KR); KIM, Un, Suwon-si Gyeonggi-do 16677 (KR); KIM, Jongsuk, Suwon-si Gyeonggi-do 16677 (KR); LEE, Yoondo, Suwon-si Gyeonggi-do 16677 (KR); LEE, Hanbin, Suwon-si Gyeonggi-do 16677 (KR); CHOE, Jaewon, Suwon-si Gyeonggi-do 16677 (KR)
(74) Representative: HGF
(86) International application number: PCT/KR2023/006920
(87) International publication number: WO 2024/014693

(57) **Abstract**

This wearable device comprises: a frame which forms at least a part of the external appearance of the wearable device; a printed circuit board (PCB) which is disposed in an inner space defined by the frame and includes a conductive pattern; a flexible PCB (FPCB) which includes a conductive portion connected to the conductive pattern and acquires an input through a pressure applied by a key button; an antenna which includes the frame, the conductive pattern, and the conductive portion; and a wireless communication circuit which is electrically connected to the antenna. The conductive portion is electrically connected to the conductive pattern. Other embodiments may also be possible.

## Description

### [Technical Field]

The present disclosure relates to a wearable device comprising a printed circuit board including a conductive pattern, and an electronic device.

### [Background Art]

With the development of technologies and the demand of users, portability is increased and electronic devices including multiple functions are required. In order to improve portability, portable electronic devices are being miniaturized. With the miniaturization of electronic devices, portable devices wearable on a body part are being provided. With the increasing demand for wearable devices such as a watch in addition to a smartphone, the wearable devices are providing electronic components to perform multiple functions in a small space.

### [Disclosure]

### [Technical Solution]

According to an embodiment, a wearable device may comprise a frame, a printed circuit board (PCB), a flexible PCB (FPCB), and wireless communication circuitry. According to an embodiment, the PCB may be disposed in an inner space surrounded by a frame and include a conductive pattern, the frame forming at least a portion of an exterior of the wearable device. According to an embodiment, the FPCB may include a conductive portion connected to the conductive pattern, and obtain an input by pressing of a key button. According to an embodiment, the wearable device may comprise an antenna using the frame, the conductive pattern, and the conductive portion as a radiator, and wireless communication circuitry electrically connected to the antenna. According to an embodiment, the conductive portion of the FPCB may be electrically connected to the conductive pattern.

According to an embodiment, an electronic device may include a housing including a first plate, a second plate facing the first plate, and a lateral frame disposed between the first plate and the second plate and including a conductive portion. According to an embodiment, the electronic device may further include a PCB disposed in the housing and including a conductive pattern. According to an embodiment, the electronic device may further include a key button inserted into an opening formed in the lateral surface. According to an embodiment, the electronic device may further include an FPCB including a conductive portion connected to the conductive pattern and a signal line configured to contact a portion of the key button and obtain an input signal through movement of the key button. According to an embodiment, the electronic device may further include a bracket electrically connected to a ground portion of the PCB and disposed between the printed circuit board and the first plate. According to an embodiment, the electronic device may further include an antenna using the lateral frame, the conductive pattern, and the conductive portion of the FPCB as an antenna radiator. The electronic device may include wireless communication circuitry electrically connected to the antenna. According to an embodiment, the conductive portion may be electrically connected to the conductive pattern.

### [Description of the Drawings]

FIG. 1 is a block diagram of an electronic device in a network environment according to various embodiments.
FIG. 2A is a perspective view of a front side of an electronic device according to an embodiment.
FIG. 2B is a perspective view of a rear side of the electronic device of FIG. 2A.
FIG. 3 is an exploded perspective view of the electronic device of FIG. 2A.
FIG. 4 illustrates an example of an internal arrangement of an electronic device according to an embodiment.
FIG. 5 is a cross-sectional view of an exemplary electronic device according to an embodiment.
FIG. 6 illustrates an exemplary power supply path of an electronic device according to an embodiment.
FIG. 7 is a graph exemplarily illustrating performance of an electronic device according to an embodiment.
FIG. 8 illustrates an exemplary connection of an FPCB of an electronic device according to an embodiment.
FIG. 9 illustrates an example of a printed circuit board according to an embodiment.
FIG. 10 illustrates an example of an FPCB of an electronic device according to an embodiment.
FIG. 11 is a graph exemplarily illustrating performance of an antenna according to an embodiment.

### [Mode for Invention]

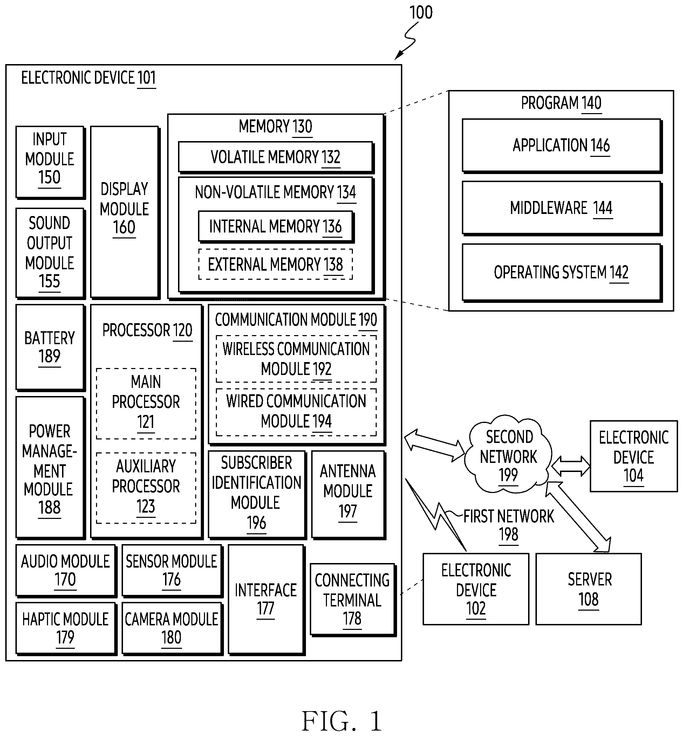

FIG. 1 is a block diagram illustrating an electronic device 101 in a network environment 100 according to various embodiments.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

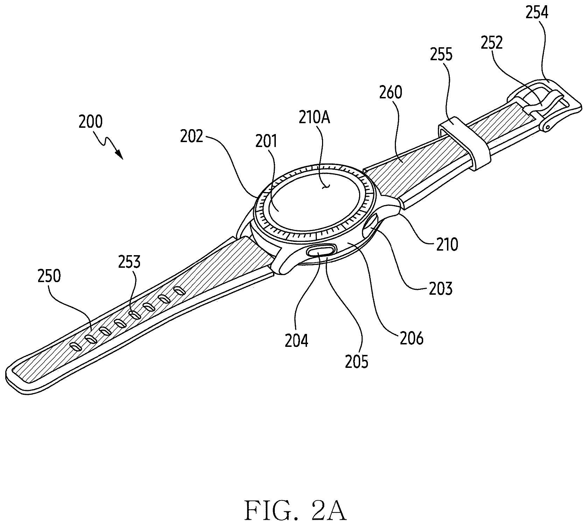

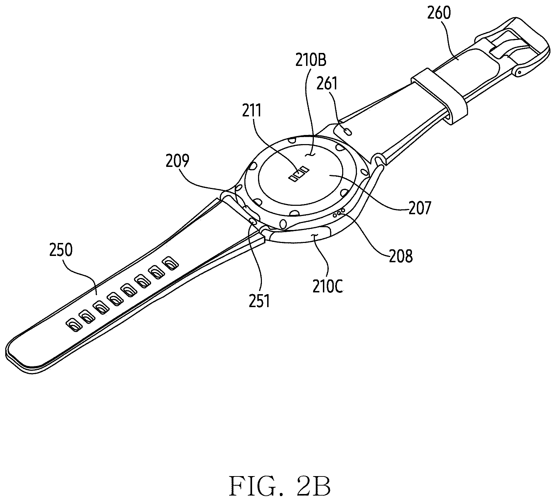

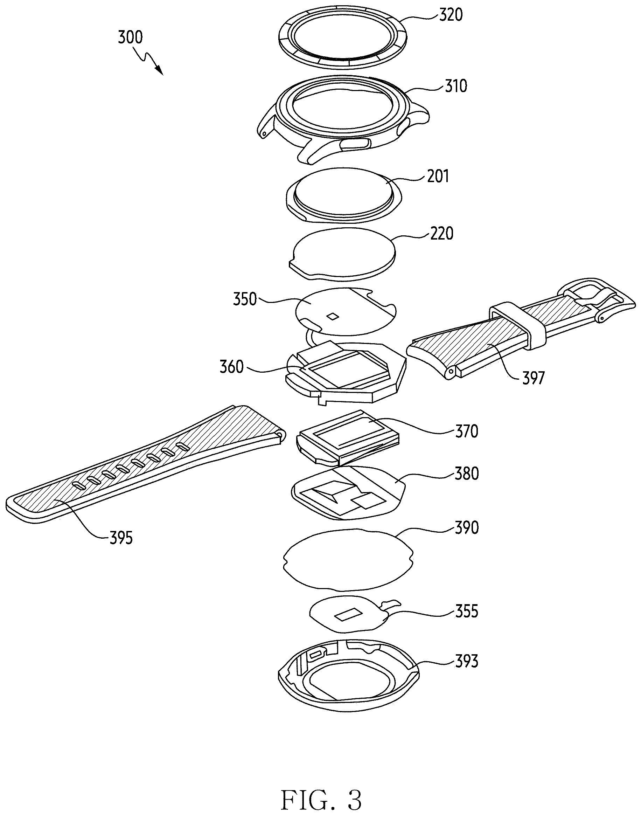

FIGS. 2A is a perspective views of a front side of an electronic device according to an embodiment. FIGS. 2B is a perspective views of a rear side of the electronic device of FIG. 2A. FIG. 3 is an exploded perspective view of the electronic device of FIG. 2A.

Referring to FIGS. 2A and 2B, an electronic device 200 (e.g., the electronic device 101 of FIG. 1) according to an embodiment may include a housing 210 including a first surface (or front surface) 210A, a second surface (or rear surface) 210B, and a side surface 210C surrounding the space between the first surface 210A and the second surface 210B and binding members 250 and 260 connected to at least a part of the housing 210 and configured to detachably attach the electronic device 200 to a part of the user's body (e.g., wrist, ankle, etc.). In another embodiment (not illustrated), the housing may also refer to a structure that forms at least a part of the first surface 210A, the second surface 210B, and the side surface 210C of FIG. 2A. According to an embodiment, at least a part of the first surface 210A may be implemented by a substantially transparent front plate 201 (e.g., glass plate or polymer plate including various coating layers). The second surface 210B may be implemented by a substantially opaque rear plate 207. The rear plate 207 may be made of, for example, coated or colored glass, ceramic, polymer, metal (e.g., aluminum, stainless steel (STS), or magnesium), or a combination of at least two of the materials. The side surface 210C may be coupled to the front plate 201 and the rear plate 207 and may be implemented by a side bezel structure (or "side member") 206 including metal and/or polymer. In some embodiments, the rear plate 207 and the side bezel structure 206 may be integrated together and may include the same material (e.g., metal material such as aluminum). The binding members 250 and 260 may be made of various materials and may be made in various shapes. The binding members 250 and 260 may be made of woven fabric, leather, rubber, urethane, metal, ceramic, or a combination of at least two of the materials.

According to an embodiment, the electronic device 200 may include at least one of a display 220 (see FIG. 3), an audio module 205 and 208, a sensor module 211, a key input device 202, 203 and 204, and a connector hole 209. In some embodiments, the electronic device 200 may omit at least one of the components (e.g., the key input devices 202, 203 and 204, the connector hole 209, or the sensor module 211) or may further include another component.

The display 220 may be exposed, for example, through a substantial portion of the front plate 201. The shape of the display 220 may correspond to the shape of the front plate 20, such as circular (shown in FIG. 2), oval, or polygonal. The display 220 may be coupled to or adjacent to a touch sensing circuit, a pressure sensor capable of measuring the strength (pressure) of touches, and/or a fingerprint sensor.

The audio modules 205 and 208 may include a microphone hole 205 and a speaker hole 208. Corresponding to the microphone hole 205, a microphone for obtaining external sound may be disposed inside, and in some embodiments, a plurality of microphones may be disposed to detect the direction of the sound. The speaker hole 208 may be used with an external speaker and a receiver for phone calls. In some embodiments, the speaker hole 208 and the microphone hole 205 may be implemented as a single hole, or a speaker (e.g., piezo speaker) may be included without the speaker hole 208.

The sensor module 211 may generate electrical signal(s) or data value(s) corresponding to internal operating state(s) of the electronic device 200 or external environmental state(s). The sensor module 211 may include, for example, a biometric sensor module 211 (e.g., heart-rate monitor (HRM) sensor) disposed on the second surface 210B of the housing 210. The electronic device 200 may further include at least one sensor module not shown, such as a gesture sensor, a gyro sensor, a pressure sensor, a magnetic sensor, an acceleration sensor, a grip sensor, a color sensor, an infrared sensor, a biometric sensor, a humidity sensor, and/or an illumination sensor.

The key input devices 202, 203, and 204 may include a wheel key 202 disposed on the first surface 210A of the housing 210 and rotatable in at least one direction, and/or side key buttons 203 and 204 disposed on the side surface 210C of the housing 210. The wheel key may have a shape corresponding to the shape of the front plate 201. In another embodiment, the electronic device 200 may not include some or all of the above-described key input devices 202, 203, and 204, and the not included key input devices 202, 203, and 204 may be implemented in other forms such as soft keys on the display 220. The connector hole 209 may accommodate a connector (e.g., USB connector) for transmitting and receiving power and/or data to and from external electronic devices and may include another connector hole (not illustrated) capable of accommodating a connector for transmitting and receiving audio signals to and from an external electronic device. The electronic device 200 may further include, for example, a connector cover (not illustrated) that covers at least a part of the connector hole 209 and blocks the inflow of external foreign material into the connector hole.

The binding members 250 and 260 may be detachably attached to at least a part of the housing 210 using locking members 251, 261. The binding members 250 and 260 may include one or more of a fixing member 252, a fixing member fastening hole 253, a band guide member 254, and a band fixing ring 255.

The fixing member 252 may be configured to fix the housing 210 and the binding members 250 and 260 to a part of the user's body (e.g., wrist, ankle, etc.). The fixing member fastening hole 253 may correspond to the fixing member 252 to fix the housing 210 and the binding members 250 and 260 to the part of the user's body. The band guide member 254 may be configured to limit movement range of the fixing member 252 when the fixing member 252 is fastened to the fixing member fastening hole 253, so that the binding members 250 and 260 are attached to be in close contact with the part of the user's body. The band fixing ring 255 may limit the range of movement of the fixing members 250 and 260 when the fixing member 252 and the fixing member fastening hole 253 are fastened.

Referring to FIG. 3, an electronic device 300 (e.g., the electronic device 101 of FIG. 1, the electronic device 200 of FIG. 2A and/or FIG. 2B) may include a side bezel structure 310, a wheel key 320, a front plate 201, a display 220, a first antenna 350, a second antenna 355, a support member 360 (e.g., bracket), a battery 370, a printed circuit board 380, a sealing member 390, a rear plate 393, and binding members 395 and 397. At least one of the components of the electronic device 300 may be the same as or similar to at least one of the components of the electronic device 200 of FIGS. 2A, and/or 2B, and repeated description thereof will be omitted. The support member 360 may be disposed inside the electronic device 300 to be connected to the side bezel structure 310 or may be integrated with the side bezel structure 310. The support member 360 may be made of, for example, metal material and/or non-metal (e.g., polymer) material. With respect to the support member 360, the display 220 may be coupled to one surface and the printed circuit board 380 may be coupled to the other surface. A processor, a memory, and/or an interface may be mounted on the printed circuit board 380. The processor may include, for example, one or more of a central processing unit, an application processor, a graphic processing unit (GPU), an application processor sensor processor, or a communication processor.

The memory may include, for example, a volatile memory or a nonvolatile memory. The interface may include, for example, a high-definition multimedia interface (HDMI), a universal serial bus (USB) interface, an SD card interface, and/or an audio interface. The interface may electrically or physically connect the electronic device 300 to an external electronic device, for example, and may include a USB connector, an SD card/MMC connector, or an audio connector.

The battery 370 is a device for supplying power to at least one component of the electronic device 300, and may include, for example, a non-rechargeable primary battery, a rechargeable secondary battery, or a fuel battery. At least a part of the battery 370 may be disposed on substantially the same plane as, for example, the printed circuit board 380. The battery 370 may be integrally disposed inside the electronic device 200 or may be disposed in the electronic device 200 to be user detachable.

The first antenna 350 may be disposed between the display 220 and the support member 360. The first antenna 350 may include, for example, a near field communication (NFC) antenna, a wireless charging antenna, and/or a magnetic secure transmission (MST) antenna. The first antenna 350 may, for example, perform short-range communication with an external device, wirelessly transmit and receive power required for charging, and transmit short-range communication signals such as ones containing payment data. In another embodiment, an antenna structure may be formed by at least a portion of the side bezel structure 310 and/or a part of the support member 360 or a combination thereof.

The second antenna 355 may be disposed between the printed circuit board 380 and the rear plate 393. The second antenna 355 may include, for example, a near field communication (NFC) antenna, a wireless charging antenna, and/or a magnetic secure transmission (MST) antenna. For example, the second antenna 355 may perform short-range communication with an external device, wirelessly transmit and receive power required for charging, and may transmit a short-range communication signal such as ones containing payment data. In another embodiment, an antenna structure may be formed by at least a portion of the side bezel structure 310 and/or a part of the rear plate 393 or a combination thereof.

The sealing member 390 may be positioned between the side bezel structure 310 and the rear plate 393. The sealing member 390 may be configured to block moisture and foreign material flowing into the space surrounded by the side bezel structure 310 and the rear plate 393 from the outside.

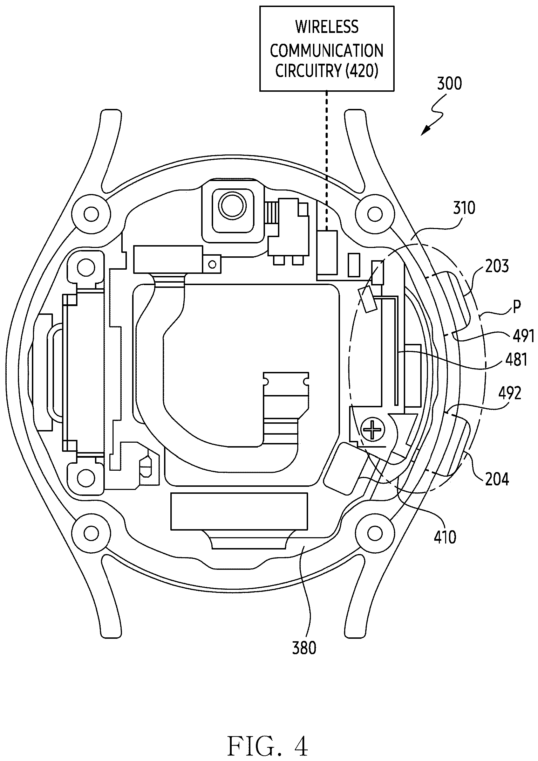

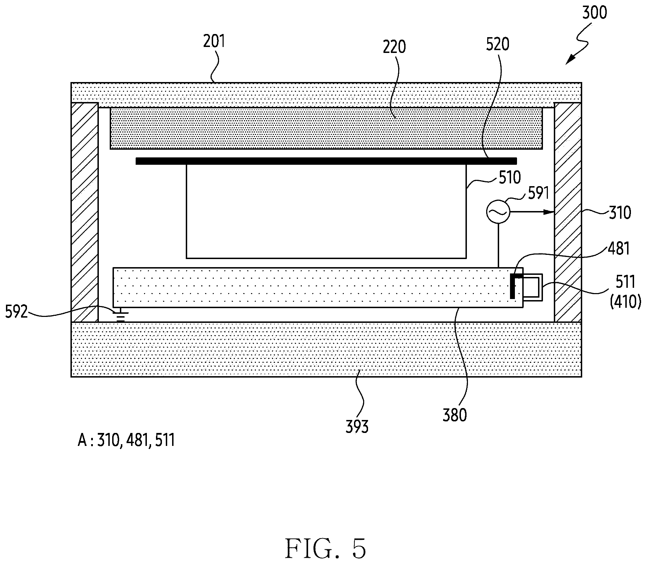

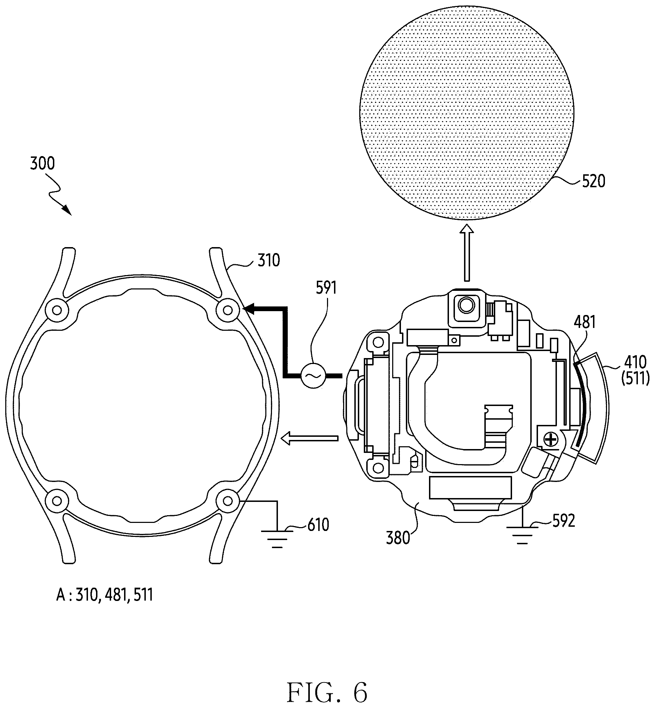

FIG. 4 illustrates an example of an internal arrangement of an electronic device according to an embodiment. FIG. 5 is a cross-sectional view of an exemplary electronic device according to an embodiment. FIG. 6 illustrates an exemplary power supply path of an electronic device according to an embodiment.

Referring to FIGS. 4 and 5, an electronic device 300 (e.g., the electronic device 101 of FIG. 1, the electronic device 200 of FIG. 2A, or the electronic device 300 of FIG. 3) may include a frame 310, a printed circuit board (PCB) 380, a FPCB 410, and wireless communication circuitry 420.

According to an embodiment, the frame 310 may form at least a portion of an exterior of the electronic device 300. The frame 310 may define an inner space of the electronic device 300. The inner space may be a space surrounded by the frame 310, a front plate (e.g., the front plate 201 of FIG. 2A), and a rear plate (e.g., the rear plate 393 of FIG. 3).

The PCB 380 may be disposed in the inner space defined by the frame 310. A conductive pattern 481 may be formed in the PCB 380. In an embodiment, the conductive pattern 481 may operate as a portion of an antenna radiator that provides a signal of a designated frequency band. For example, the conductive pattern 481 may operate as an antenna radiator together with a conductive portion of the frame 310. For example, the conductive pattern 481 and the frame 310 may be configured to communicate a signal in a low band or a middle band with an external electronic device. According to an embodiment, the antenna A may use the conductive pattern 481 and the conductive portion of the frame 310 as an antenna radiator. The antenna A using the conductive pattern 481 and the frame 310 as an antenna radiator may communicate with an external electronic device through the wireless communication circuitry 420.

According to an embodiment, the FPCB 410 may include a conductive portion 511 electromagnetically connected to the conductive pattern 481, and may obtain an input by pressing a key button 203 or 204. For example, the conductive portion 511 may include a ground of the FPCB 410. The conductive portion 511 of the FPCB 410 may provide a ground path. The conductive portion 511 may provide a ground path distinct from a signal line in the FPCB 410. For example, the conductive portion 511 may be coupled to the frame 310, and at least a portion thereof may be used as a radiator together with the conductive pattern 481. For example, an end of the FPCB 410 may be electrically connected to a portion of the conductive pattern 481. Another end of the FPCB 410 may be connected to a ground portion of the PCB 380. The wireless communication circuitry 420 may be configured to communicate with an external electronic device through at least a portion of the frame 310, the conductive pattern 481, and the conductive portion 511 in the FPCB 410. For example, the wireless communication circuitry 420 may provide a power supply signal to the conductive pattern 481 or the frame 310 for communication with an external electronic device.

According to an embodiment, the FPCB 410 may be disposed between an inner surface of the frame 310 and a lateral surface of the PCB 380. In an embodiment, the frame 310 may include openings 491 and 492. For example, the openings 491 and 492 may overlap a portion of the FPCB 410 when the FPCB is viewed from above. Key buttons 203 and 204, inserted into the opening 491 and 492, configured to translate may be positioned in the frame. The FPCB 410 may provide an input signal by pressing at least one of the key buttons 203 and 204. For example, when pressing the key buttons 203 and 204, the FPCB 410 may generate an input signal through a dome key (not illustrated in FIGS. 4, 5, and 6) disposed on the FPCB 410. The FPCB 410 may transmit the generated input signal to a processor (e.g., the processor 120 of FIG. 1), which is disposed on the PCB 380 or electrically connected thereto. According to an embodiment, the key buttons 203 and 204 may include a conductive material. The key buttons 203 and 204 may be connected to a conductive line (e.g., signal line) distinct from the conductive portion in the FPCB to obtain a biometric signal through a microcurrent obtained through the conductive material. For example, the key buttons 203 and 204 may transmit a microcurrent supplied from a body part of a user in contact with the key buttons 203 and 204 to the FPCB 410 through the conductive material. The microcurrent transmitted to the FPCB 410 may transmit data related to the microcurrent to the processor 120 through the conductive line. For example, the FPCB 410 may include a biometric sensor capable of obtaining electrical signals related to electrocardiogram (ECG) or bioelectrical impedance analysis (BIA) data, or may be electrically connected to the biometric sensor. The biometric sensor, which is a BIA sensor, may measure the amount of muscle mass, fat, and moisture in the body by flowing a micro current into the body, based on the degree of passage of electricity according to the amount of water in the body. The biometric sensor, which is an ECG sensor, may obtain a blood vessel activity potential difference according to the heartbeat through an ECG electrode. In an embodiment, the FPCB 410 may include a conductive portion 511 for grounding the biometric sensor, and the conductive portion 511 may operate as an antenna radiator together with the conductive pattern 481. The antenna A may use the conductive portion 511 and the conductive pattern 481 as an antenna radiator.

According to an embodiment, the FPCB 410 may be substantially perpendicular to a surface of the PCB 380. For example, the FPCB 410 may be disposed along a portion of a lateral surface perpendicular to the surface of the PCB 380. The FPCB 410 may be disposed between the conductive pattern 481 and the frame 310. For example, the FPCB 410 may be disposed along a portion of an edge adjacent to the conductive pattern 481 included in the PCB 380. The FPCB 410 may be disposed along an inner surface of the frame 310 facing a portion of the conductive pattern 481. For example, the conductive pattern 481 and the frame 310 may be disposed physically close to the FPCB 410. In an embodiment, the conductive pattern 481 and the frame 310 may be coupled to the conductive portion 511 of the FPCB 410. For example, the conductive portion 511 of the FPCB 410, the frame 310, and the conductive pattern 481 may be coupled to each other and operate as an antenna radiator. For example, the conductive portion 511, the frame 310, and the conductive pattern 481 within a region P in which an electric field is strongly formed in a designated frequency band may be coupled to each other. The antenna A may use the frame 310, the conductive pattern 481, and the conductive portion 511 as an antenna radiator.

Referring to FIGS. 5 and 6, the frame 310 may receive a wireless communication signal to be transmitted to an external electronic device through the wireless communication circuitry 420 and a power supply unit 591. In an embodiment, the electronic device 300 may include a bracket 520. For example, the bracket 520 may be electrically connected to a ground portion of the PCB 380 and disposed between the PCB 380 and the display 220. In an embodiment, the bracket 520 may include a conductive material. For example, the bracket 520 may be connected to a ground portion of the PCB 380 or a ground 592 of the electronic device 300. The bracket 520 may provide a ground to the PCB 380. In an embodiment, the conductive portion 511 in the FPCB 410 may be electrically connected to the ground portion of the PCB 380.

According to an embodiment, the bracket 520 may be a structure supporting the display 220 and may include a conductive material. For example, the bracket 520 may be disposed between the display 220 and a battery 510. For example, the battery 510 may be supported by the bracket 520 and attached to a portion of the bracket 520. For example, the bracket 520 may support not only the display 220 and the battery 510 but also another electronic component disposed inside the electronic device 300. In an embodiment, the bracket 520 may include a conductive portion and be connected to a ground line of the PCB 380 to provide a ground to the PCB 380. According to an embodiment, the frame 310 may operate as a ground 610.

According to an embodiment, a sum of a length of the conductive pattern 481 and a length of the conductive portion 511 may be 1/4 of a wavelength of an electromagnetic wave including a signal in communication with the external electronic device. For example, in order to compensate for a lack of a resonance length of the frame 310, the conductive pattern 481 and the conductive portion 511 may be used as an antenna radiator. For example, when transmitting a signal in the low-band or the middle-band to the outside, a relatively long length of the antenna radiator may be required for resonance. In a case of a small wearable device, a resonance length of the frame therein may be insufficient. For the insufficient resonance length, an additional conductive pattern may be required. In order to provide a conductive pattern or a conductive portion, an additional antenna (e.g., a laser direct structuring antenna (LDS)) or a separate conductive portion may be required. When a separate antenna component is added, a mounting space may be required inside the electronic device 300. By utilizing the conductive portion 511 for grounding the FPCB 410 inside the electronic device 300, the electronic device 300 may save a mounting space of an additional component.

According to an embodiment, the conductive portion 511 of the FPCB 410 may be electrically connected to the conductive pattern 481. For example, the conductive portion 511 may be electrically connected to the conductive pattern 481 through a conductive structure (e.g., contact or c-clip) that is disposed on the PCB 380 and has elasticity. According to an embodiment, the conductive portion 511 may be used as an antenna radiator together with the frame 310 and the conductive pattern 481.

According to an embodiment, the conductive portion 511 and/or the conductive pattern 481 may be coupled to the frame 310. For example, the conductive portion 511 faces the frame 310 and may be electrically connected to the conductive pattern 481. The conductive portion 511, the frame 310, and the conductive pattern 481, which are coupled, may be used as an antenna radiator to ensure a relatively insufficient resonance length.

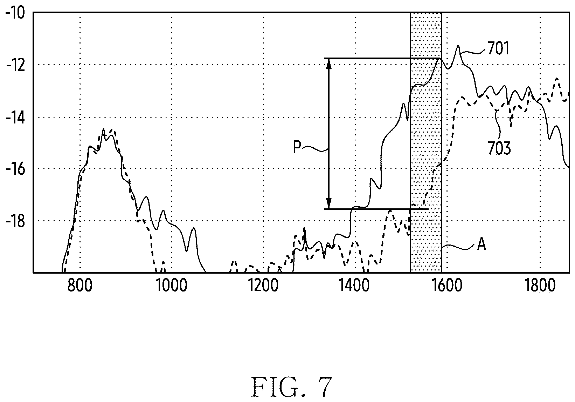

FIG. 7 is a graph exemplarily illustrating performance of an electronic device according to an embodiment.

Referring to FIG. 7, a graph 701 represents antenna performance of the electronic device 300 according to an embodiment, and a graph 703 represents antenna performance of a comparative electronic device. The x-axis represents a frequency of a signal, and the y-axis relatively represents magnitude of the signal. A unit on the x-axis may be KHz, and a unit on the y-axis may be dB.

The graph 701 represents a gain according to a frequency when the conductive pattern 481 and the conductive portion 511 are connected to each other to additionally use the conductive pattern 481 and the conductive portion 511 as an antenna radiator.

The graph 703 represents a gain according to a frequency when a conductive pattern is not included, differently from the graph 701. For example, a conductive portion of the FPCB 410 may not be utilized as a portion of a radiator and may operate as a ground.

Referring to the graph 701 and the graph 703, a resonance length of a GPS band A may be secured through the conductive pattern 481 electrically connected to the conductive portion 511, thereby improving performance of a GPS antenna. For example, the frame 310, the conductive portion 511 and the conductive pattern 481 may secure a resonance length in the GPS band A, which is approximately 1.5 GHz to 1.6 GHz, and increase an antenna gain by approximately P compared to a reference electronic device.

According to the above-described embodiment, a resonance length may be secured by electrically connecting the conductive portion 511 of the FPCB 410 and the conductive pattern 481 of the PCB 380 to provide 1/4 of a wavelength of an electromagnetic wave of a signal communicating with an external electronic device. By securing the resonance length, antenna performance in a low frequency band or a middle frequency band may be improved.

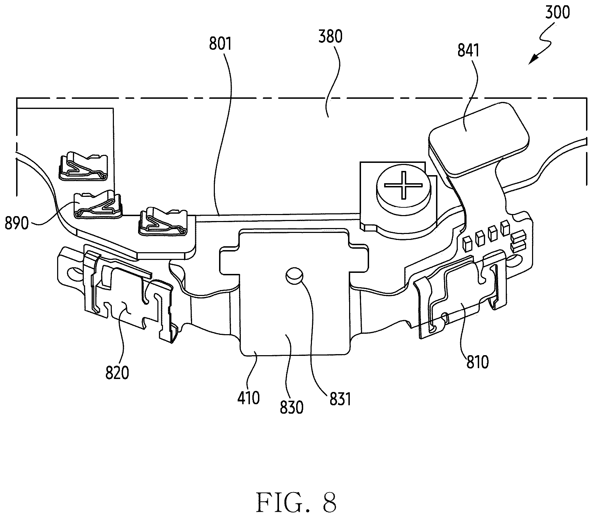

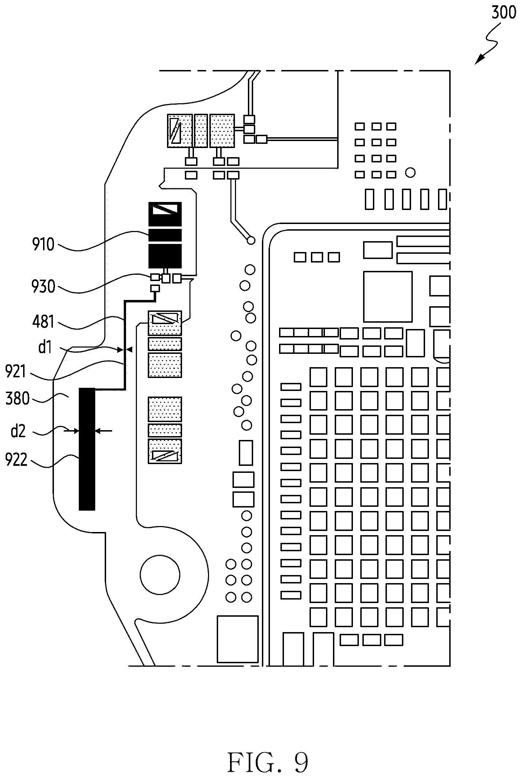

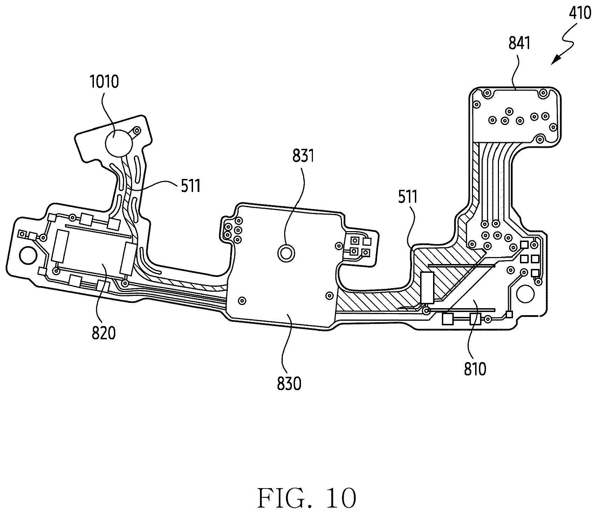

FIG. 8 illustrates an exemplary connection of an FPCB of an electronic device according to an embodiment. FIG. 9 illustrates an example of a printed circuit board according to an embodiment. FIG. 10 illustrates an example of an FPCB of an electronic device according to an embodiment.

Referring to FIGS. 8, 9, and 10, a FPCB 410 may be disposed along a portion of an edge of a PCB 380. The FPCB 410 may be electrically connected to the PCB 380 through a connector 841 or a connection member 890 with elasticity. For example, a conductive portion 511 of the FPCB 410 may be electrically connected to the conductive pattern 481 through the connection member 890, which is a conductive structure disposed between the PCB 380 and the FPCB 410 and has elasticity. For example, an end of the FPCB 410 may be electrically connected to a portion of the conductive pattern 481. For example, an end of the FPCB 410 and the conductive pattern 481 may be electrically connected through the connection member 890 with elasticity. For example, the connection member 890 may be a contact or a C-clip. According to an embodiment, the FPCB 410 may further include a connector 841 connecting the conductive portion 511 of the FPCB 410 and the PCB 380 at another end of the FPCB 410. The other end of the FPCB 410 may be connected to a ground portion of the PCB 380. A conductive portion 511 (e.g., the conductive portion 511 of FIG. 5) of the FPCB 410 may be connected to the ground portion of the PCB 380 through the connector 841.

According to an embodiment, a conductive portion 511 (e.g., the conductive portion 511 of FIG. 5) of the FPCB 410 may be connected to the ground portion of the PCB 380 through the connector 841, and a signal line distinct from the conductive portion 511 may also be connected to a line of the PCB 380 through the connector 841.

According to an embodiment, the FPCB 410 may be configured to identify the pressing of a key button (e.g., key buttons 203 and 204 of FIG. 2A). The FPCB 410 may include dome keys 810 and 820 disposed in a region in contact with the key buttons 203 and 204 for identifying the pressing. The dome keys 810 and 820 may be elastically pressed according to movement of the key buttons 203 and 204. For example, the dome keys 810 and 820 may enable the electronic device 300 to perform a designated function (e.g., switching to a home screen or returning to a previous screen) when pressed.

According to an embodiment, the key buttons 203 and 204 may include a conductive material. The key buttons 203 and 204 may be connected to a conductive line distinct from the conductive portion 511 in the FPCB 410 to obtain a biometric signal through a microcurrent obtained through the conductive material. The dome keys 810 and 820 in contact with the key buttons 203 and 204 may include a conductive material. The conductive material of the key buttons 203 and 204 may be an electrode and may be electrically connected to the dome keys 810 and 820. The biometric signal obtained through the key buttons 203 and 204 may transmit the obtained biometric signal to a processor (e.g., the processor 120 of FIG. 1) through a signal line electrically connected to the dome keys 810 and 820 or the key buttons 203 and 204. The key buttons 203 and 204 may include an ECG electrode or a BIA electrode. For example, a key button 203 may include the ECG electrode, and another key button 204 may include the BIA electrode. However, it is not limited thereto, and one of the key buttons 203 and 204 may perform a plurality of functions. The FPCB 410 may be electrically connected to the ECG electrode or the BIA electrode. For example, a key button 203 may include the ECG electrode and the BIA electrode. Another key button 204 may include the BIA electrode. When ECG data is obtained to measure a heart rate, an external object (e.g., a user's finger) may be contacted to the key button 203. In response to a signal requesting obtainment of the ECG data, an ECG sensor (not illustrated) connected to the key button 203 may obtain a biometric signal through the key button 203. For another example, when BIA data is obtained to measure body fat, external objects may be contacted to each of the key buttons 203 and 204. In response to a signal requesting obtainment of the BIA data, a BIA sensor (not illustrated) connected to the key buttons 203 and 204 may obtain other biometric signals different from the biometric signal through the key buttons 203 and 204.

According to an embodiment, the FPCB 410 may include a hole 831 corresponding to a microphone hole of the frame (e.g., the frame 310 of FIG. 4). The hole 831 may be formed in an arrangement region of the microphone 830 in the FPCB 410. The FPCB 410 may be electrically connected to the microphone 830 to transmit an audio signal transmitted from the microphone hole through the microphone 830 disposed in the electronic device. The microphone 830 may be disposed in the FPCB 410. The microphone 830 may be disposed in the FPCB 410, but is not limited thereto. For example, the microphone 830 may be disposed within the electronic device 300, and receive an audio signal through the hole 831.

According to an embodiment, the FPCB 410 may be disposed along a portion of a lateral surface 801 of the PCB 380. The conductive portion 511 in the FPCB 410 may be coupled to the frame 310.

Referring to FIGS. 9 and 10, an example of a printed circuit board according to an embodiment is illustrated.

According to an embodiment, the PCB 380 may include a conductive portion 910 electrically connected to a connection member 890 (e.g., the connection member 890 of FIG. 8). For example, the conductive portion 910 may be electrically connected to the connection member 890, and electrically connect the conductive portion 511 of the FPCB 410 and the conductive pattern 481 of the PCB 380.

According to an embodiment, the FPCB 410 may include a conductive pad 1010 in contact with the connection member 890. The conductive pad 1010 may be electrically connected to the conductive portion 511. For example, the conductive portion 511 may be a ground path. For example, a portion of the conductive portion 511, which is electrically connected to the conductive pattern 481 through the conductive pad 1010, may be utilized as an antenna radiator. The conductive portion 511 may be electrically connected to the dome keys 810 and 820, the microphone 830, and the connector 841. For example, the conductive portion 511 may be extended from the conductive pad 1010 to the connector 841 via the dome key 820, the microphone 830, and the dome key 810, and be connected to a ground portion of the PCB 380.

According to an embodiment, the PCB 380 may further include impedance matching circuitry 930. The impedance matching circuitry 930 may include switching elements and/or passive elements such as an inductor or a capacitor. The impedance matching circuitry 930 may tune the impedance for resonance of the conductive pattern 481, the conductive portion 511 of the FPCB 410, and the frame 310, which are utilized as a radiator, through the passive element and the switching element.

According to an embodiment, the conductive pattern 481 may be electrically connected to the impedance matching circuitry 930 adjacent to the conductive portion 910, which is a region connected to an end of the FPCB 410. For example, the impedance matching circuitry 930 may be disposed between the conductive pattern 481 and the connection member 890. The impedance matching circuitry 930 may be electrically connected to the conductive pattern 481 and the connection member 890.

According to an embodiment, the conductive pattern 481 may extend along a portion of an edge (e.g., the lateral surface 801) of the PCB 380. The conductive pattern 481 may include, on the PCB 380, a region 922 (e.g., a second region) having a width different from that of a region 921 (e.g., a first region) connected to the impedance matching circuitry 930. For example, a width d1 of the first region 921 of the conductive pattern 481 connected to the impedance matching circuitry 930 may be narrower than a width d2 of the second region 922.

According to the embodiment, the width d2 of the second region 922 extending from the first region 921 may be approximately 1.5mm to 2.5mm. In an embodiment, the width of the second region 922 may be determined based on a frequency of an antenna including the conductive pattern 481. For example, in case that an antenna including the conductive pattern 481 supports a GPS band, a width of the region 920 extending along a surface of the FPCB may be approximately 2mm based on an impedance matching value for each frequency.

According to an embodiment, a sum of a length of the conductive pattern 481 and a length of the conductive portion 511 may be 1/4 of a wavelength of an electromagnetic wave including a signal communicating with the external electronic device. For example, a sum of a length of the conductive pattern 481 disposed on the PCB 380 and a length of the conductive portion 511 of the FPCB 410 may be approximately 50mm to improve frequency performance in approximately 1.5 GHz band.

According to the above-described embodiment, a conductive portion connected to a ground portion disposed inside the electronic device 300 may be utilized as an antenna radiator to secure an insufficient resonance length. For example, the conductive portion, which is a ground path in the FPCB 410 that is a key flexible printed circuit board providing an input signal by contacting with a key button, may be utilized as an antenna radiator to save a mounting space for arranging the antenna radiator. A component within the electronic device 300 may be utilized to solve a problem of insufficient resonance length, and a product manufacturing cost may be reduced.

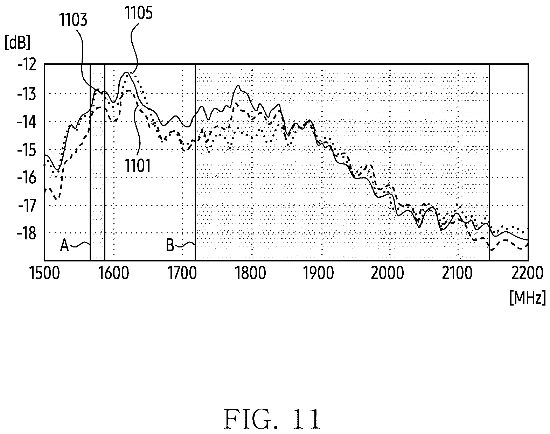

FIG. 11 is a graph exemplarily illustrating performance of an antenna according to an embodiment.

Referring to FIG. 11, a graph 1101, a graph 1103, and a graph 1105 represent antenna performance according to a width of the region 920 of the conductive pattern 481. The x-axis represents a frequency of a signal, and the y-axis relatively represents magnitude of the signal. A unit on the x-axis may be KHz, and a unit on the y-axis may be dB.

The graph 1101 represents antenna performance when the width d2 of the second region 922 of the conductive pattern 481 is 0.5 mm. The graph 1103 represents antenna performance when the width d2 of the second region 922 of the conductive pattern 481 is 2 mm. The graph 1105 represents antenna performance when the width d2 of the second region 922 of the conductive pattern 481 is 2.5 mm.

The extended region 920 of the conductive pattern 481 may be a region coupled to the conductive portion 511 of the FPCB 410. When the width d2 of the extended second region 922 of the conductive pattern 481 increases, the amount of coupling due to the bandwidth expansion may increase. As the width d2 of the extended second region 922 of the conductive pattern 481 is changed, the antenna performance may be changed. For example, when looking at the graph 1103 and the graph 1105 in the GPS band A due to an increase in the amount of coupling, antenna performance may be improved due to an increase in the width d2 of the second region 922. For example, when looking at the graph 1103 and the graph 1105, due to the increase in the amount of coupling, the antenna performance due to the increase in the width d2 of the second region 922 may be improved in the GPS band A. For example, as described above, a width of the region 920 may be determined according to a frequency band. The width of the region 920 may be determined as a width advantageous for impedance matching at 2.0 mm or 2.5 mm. For example, the width d2 of the second region 922, which is determined based on the GPS band A, may be 2.0 mm. In another band B distinct from the GPS band A, correlation between antenna performance and width may be small.

According to the above-described embodiment, the conductive pattern 481 may increase the amount of coupling according to the width d2 of the second region 922. By increasing the width of the conductive pattern 481, the amount of coupling with the conductive portion 511 of the FPCB 410 may be increased. Based on the increased coupling amount, the antenna performance may be changed.

According to the above-described embodiment, a wearable device (e.g., the electronic device 100 of FIG. 1, the electronic device 200 of FIG. 2A, or the electronic device 300 of FIG. 3) may include a frame (e.g., the frame 310 of FIG. 3). The frame may surround at least a portion of an exterior of the wearable device. The wearable device may further include a printed circuit board (e.g., the PCB 380 of FIG. 3). The printed circuit board may be disposed in an inner space distinguished by the frame. The printed circuit board may include a conductive pattern (e.g., the conductive pattern 481 of FIG. 4). The wearable device may further include an FPCB (e.g., the FPCB 410 of FIG. 4). The FPCB may include a conductive portion (e.g., the conductive portion 511 of FIG. 5) connected to the conductive pattern, and may obtain an input signal through a key button (e.g., the key buttons 203 and 204 of FIG. 2A). The wearable device may further include an antenna using the frame, the conductive pattern, and the conductive portion in the FPCB as a radiator. The wearable device may further include wireless communication circuitry (e.g., the wireless communication circuitry 420 of FIG. 4). The wireless communication circuitry may be electrically connected to an antenna. According to an embodiment, the conductive portion may be electrically connected to the conductive pattern. According to the above-described embodiment, a resonance length may be secured through the FPCB connected to a conductive pattern of the printed circuit board. As the resonance length is secured, space efficiency of the wearable device may be secured. The above-described embodiment may have various effects including the above-described effects.

According to an embodiment, the wearable device may further include a bracket (e.g., the bracket 520 of FIG. 5). The bracket may be electrically connected to a ground portion of the PCB and may be disposed on the PCB. According to the above-described embodiment, the bracket may provide a ground portion by being electrically connected to the printed circuit board.

According to an embodiment, the conductive portion in the FPCB may be electrically connected to the ground portion of the PCB. According to the above-described embodiment, the FPCB may reduce a component for an antenna radiator by utilizing a conductive portion utilized as a ground path. The above-described embodiment may have various effects including the above-described effects.

According to an embodiment, the FPCB may be disposed between the inner surface of the frame and a lateral surface of the PCB. The frame may include an opening that opens a portion of a region overlapping the FPCB when the FPCB is viewed from above, and a key button, inserted into the opening, configured to translate.

According to an embodiment, the FPCB may include a dome key, configured to identify pressing of the key button, disposed in a region in contact with the key button. According to the above-described embodiment, the FPCB may be a key FPCB for a key button. A manufacturing costs may be reduced by utilizing a component disposed inside the wearable device as an antenna radiator.

According to an embodiment, the key button may include a conductive material and may be connected to a conductive line distinct from the conductive portion in the FPCB, in order to obtain a biometric signal through a microcurrent obtained through the conductive material.

According to an embodiment, the conductive portion in the FPCB may be coupled to the frame. The above-described embodiments may utilize a conductive portion that is easily coupled to a frame in the FPCB adjacent to a frame as an antenna radiator, thereby securing a resonance length and improving antenna performance.

According to an embodiment, the conductive portion of the FPCB may be electrically connected to the conductive pattern through a conductive structure disposed on the PCB and pressing the FPCB.

According to an embodiment, the FPCB may further include, in the other end, a connector (e.g., the connector 841 of FIG. 8) that connects a conductive portion (e.g., the conductive portion 511 of FIG. 5) of the FPCB and the ground portion of the PCB.

According to an embodiment, the conductive pattern may extend along a portion of the inner surface of the frame.

According to the above-described embodiment, the FPCB may be electrically connected to the conductive pattern and the ground portion on the PCB. The FPCB may be coupled to a portion of the frame by facing a portion of the frame used as an antenna radiator, and the conductive pattern of the printed circuit board may be coupled to the conductive portion of the FPCB. The frame, the conductive pattern of the printed circuit board, and the conductive portion of the FPCB, which are coupled to each other, may be utilized as an antenna radiator to communicate with an external electronic device.

The FPCB may be substantially perpendicular to a surface of the printed circuit board. The FPCB may be disposed between the conductive pattern and the frame. The conductive pattern, the frame, and the conductive portion of the FPCB may be disposed adjacent to each other to be electrically coupled to each other. By the coupling, the conductive pattern, the frame, and the conductive portion of the FPCB may operate as an antenna radiator.

A width of a region of the conductive pattern connected to an end of the FPCB may be narrower than a width of a region (e.g., the region 920 of FIG. 9) extending along a surface of the FPCB.

According to an embodiment, the region extending along a surface of the FPCB may have a width of 1.5 mm to 2.5 mm. According to the above-described embodiment, when a width of the region extending along a surface of the FPCB in the conductive pattern is increased, the amount of coupling with the FPCB may be increased. Based on the increased coupling, antenna performance may be improved.

According to an embodiment, a sum of a length of the conductive pattern and a length of the conductive portion may be 1/4 of a wavelength of an electromagnetic wave including a signal communicating with the external electronic device. Since lengths of the conductive pattern and the conductive portion are determined based on the wavelength of the electromagnetic wave, resonance may be formed in a designated frequency band.

According to an embodiment, the conductive pattern may be electrically connected to impedance matching circuitry spaced apart from a region connected to an end of the FPCB.

According to an embodiment, the key button may be an ECG electrode or a BIA electrode. The FPCB may be electrically connected to the key button including the ECG electrode or the BIA electrode.

According to an embodiment, the frame may include a microphone hole, and the FPCB may be electrically connected to a microphone to transmit an audio signal transmitted from the microphone hole through the microphone disposed in the wearable device.

According to an embodiment, an electronic device (e.g., the electronic device 300 of FIG. 3) may include a housing 210. The housing may include a first plate (e.g., the front plate 201 of FIG. 3), a second plate (e.g., the rear plate 393 of FIG. 3) facing the first plate, and a lateral frame (e.g., the frame 310 of FIG. 3) disposed between the first plate and the second plate and including a conductive portion. The electronic device may further include a PCB (e.g., the printed circuit board 390 of FIG. 3). The PCB may be disposed in the housing and may include a conductive pattern (e.g., the conductive pattern 481 of FIG. 4). The electronic device may include a key button (e.g., the key buttons 203 and 204 of FIG. 4). The key button may linearly reciprocate through an opening (e.g., the openings 491 and 492 of FIG. 4) formed in the lateral frame. The electronic device may further include an FPCB (e.g., the FPCB 410 of FIG. 4). The FPCB may be contacted with a conductive portion connected to the conductive pattern and a portion of the key button. The FPCB may include a signal line configured to obtain an input signal through movement of the key button. The electronic device may further include a bracket (e.g., the bracket 520 of FIG. 5). The bracket may be electrically connected to a ground portion of the PCB and may be disposed between the PCB and the first plate. The electronic device may include an antenna using the lateral frame 310, the conductive pattern 481, and the conductive portion 511 as a radiator. The electronic device may further include wireless communication circuitry (e.g., the wireless communication circuitry 420 of FIG. 4). The wireless communication circuitry may be electrically connected to the antenna. According to an embodiment, the conductive portion may be electrically connected to the conductive pattern. According to the above-described embodiment, a resonance length may be secured through the FPCB connected to the conductive pattern of the printed circuit board. By securing the resonance length, space efficiency of the wearable device may be secured. The above-described embodiment may have various effects including the above-described effects.

According to an embodiment, the FPCB may be disposed between an inner surface of the frame and a lateral surface of the PCB. According to an embodiment, the opening may overlap the FPCB when viewed from above.

The FPCB may include a dome key disposed at a region in contact with the key button. The FPCB may be configured to identify pressing of the key button through the dome key. According to the above-described embodiment, the FPCB may be a key FPCB for a key button. A manufacturing cost may be reduced by using a component disposed inside the wearable device as an antenna radiator.

According to an embodiment, the key button may include a conductive material. According to an embodiment, it may be connected to a conductive line distinct from the conductive portion in the FPCB to obtain a biometric signal through a microcurrent obtained through the conductive material. According to an embodiment, the conductive portion may be coupled to the frame. The above-described embodiments may utilize a conductive portion that is easily coupled to a frame in the FPCB adjacent to a frame as an antenna radiator, thereby securing a resonance length and improving antenna performance.

According to an embodiment, a conductive portion of the FPCB may be electrically connected to the conductive pattern through a conductive structure disposed on the PCB and having elasticity. The FPCB may further include a connector connecting the conductive portion of the FPCB and the ground portion of the PCB at the other end.

According to an embodiment, the conductive pattern may be electrically connected to impedance matching circuitry spaced apart from a region connected to an end of the FPCB.

According to an embodiment, the key button may include an ECG electrode or a BIA electrode, and the FPCB may be electrically connected to the ECG electrode or the BIA electrode. According to an embodiment, the frame may include a microphone hole. In order to transmit an audio signal transmitted from the microphone hole through a microphone disposed in the wearable device, the FPCB may be electrically connected to the microphone. According to the above-described embodiment, the FPCB may be a key FPCB for a key button. A manufacturing cost may be reduced by using a component disposed inside the wearable device as an antenna radiator.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," or "connected with" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between a case in which data is semi-permanently stored in the storage medium and a case in which the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable device (101; 200; 300) comprising:
a frame (310) forming at least a portion of an exterior of the wearable device (101; 200; 300);
a printed circuit board (PCB) (380), disposed in an inner space surrounded by the frame (310), including a conductive pattern (481);
a flexible PCB (FPCB) (410), including a conductive portion (511) connected to the conductive pattern (481), configured to obtain an input by pressing of a key button (203; 204);
an antenna (A) using the frame (310), the conductive pattern (481), and the conductive portion (511) as a radiator; and
wireless communication circuitry (420) electrically connected to the antenna,
wherein the conductive portion (511) is electrically connected to the conductive pattern (481).

2. The wearable device (101; 200; 300) of claim 1, further comprising a bracket (520), electrically connected to a ground portion of the PCB (380) disposed on the PCB (380).

3. The wearable device (101; 200; 300) of any one of the preceding claims,
wherein the conductive portion (511) of the FPCB (410) is electrically connected to the ground portion of the PCB (380).

4. The wearable device (101; 200; 300) of any one of the preceding claims,
wherein the FPCB (410) is disposed between an inner surface of the frame (310) and a lateral surface 801 of the PCB (380),
wherein the frame (310) includes an opening that opens a portion of a region overlapping the FPCB (410) when the FPCB (410) is viewed from above, and
wherein the key button (203; 204), inserted into the opening of the frame (310), is configured to translate.

5. The wearable device (101; 200; 300) of any one of the preceding claims,
wherein the FPCB (410) includes a dome key (810; 820), configured to identify the pressing of the key button (203; 204), disposed in a region contacted with the key button (203; 204).

6. The wearable device (101; 200; 300) of any one of the preceding claims,
wherein the key button (203; 204) includes a conductive material, and is electrically connected a conductive line distinct from the conductive portion (511) of the FPCB (410), to obtain a biometric signal obtained through microcurrent obtained through the conductive material.

7. The wearable device (101; 200; 300) of any one of the preceding claims,
wherein the conductive portion (511) of the FPCB (410) is coupled with the frame (310).

8. The wearable device (101; 200; 300) of any one of the preceding claims,
wherein the conductive portion (511) of the FPCB (410) is electrically connected to the conductive pattern (481), through a conductive structure (890) that is disposed on the PCB (380) and has an elasticity.

9. The wearable device (101; 200; 300) of any one of the preceding claims,
wherein the FPCB (410) includes a connector (841) that connects the conductive portion (511) of the FPCB (410) and a ground portion of the PCB (380).

10. The wearable device (101; 200; 300) of any one of the preceding claims,
wherein the conductive pattern (481) extends along a portion of a periphery of the PCB (380) facing an inner surface of the frame (310).

11. The wearable device (101; 200; 300) of any one of the preceding claims,
wherein the FPCB (410) is substantially perpendicular to a surface of the PCB (380), and is disposed between the conductive pattern (481) and the frame (310).

12. The wearable device (101; 200; 300) of claim 11,
wherein a width of a region of the conductive pattern (481) connected to the FPCB (410) is narrower than a width of a region of the conductive pattern (481) extending along a surface of the FPCB (410).

13. The wearable device (101; 200; 300) of claim 12,
wherein the width of the region (920) of the conductive pattern (481) extending along the surface of the FPCB (410) is 1.5mm to 2.5mm.

14. The wearable device (101; 200; 300) of any one of the preceding claims,
wherein a sum of a length of the conductive pattern (481) and a length of the conductive portion (511) corresponds to 1/4 of a wavelength of an electromagnetic wave including a signal communicating with an external electronic device.

15. The wearable device (101; 200; 300) of any one of the preceding claims,
wherein the conductive pattern (481) is electrically connected to impedance matching circuitry (930) spaced apart from a region of the conductive pattern (481) connected to the FPCB (410).
